# EUROPEAN PATENT SPECIFICATION

(11) **EP 2 091 900 B1**
(45) Date of publication and mention of the grant of the patent: **26.12.2012**
(21) Application number: 07861733.9
(22) Date of filing: 06.11.2007
(51) Int. Cl.: C07C 21/18, C07C 17/278

(54) **METHOD OF MANUFACTURE OF FLUORINATED OLEFINS**
VERFAHREN ZUR HERSTELLUNG FLUORIERTER OLEFINE
PROCÉDÉ DE FABRICATION D'OLÉFINES FLUORÉES

(30) Priority: 07.11.2006 RU 2006139128
(43) Date of publication of application: 26.08.2009
(73) Proprietor: E.I. DU PONT DE NEMOURS AND COMPANY, Wilmington, DE 19898 (US)
(72) Inventor: STEPASHKOV, Denis Mikhailovich, Moscow 115304 (RU); CHERSTKOV, Victor Filippovich, Moscow 117574 (RU)
(74) Representative: Matthews, Derek Peter
(86) International application number: PCT/US2007/023330
(87) International publication number: WO 2008/057513

(56) References cited:
- WO-A-97/02227
- US-A1- 2006 258 891
- US-B1- 6 184 426
- HASZELDINE R N ET AL: "REACTION OF HEXAFLUOROPRENE WITH HALOGENOALKANES" 2 October 1982 (1982-10-02), JOURNAL OF FLUORINE CHEMISTRY, ELSEVIER SEQUOIA, LAUSANNE, CH, PAGE(S) 253-259 , XP002067833 ISSN: 0022-1139 the whole document

## Description

### CROSS REFERENCE(S) TO RELATED APPLICATION(S)

This application claims the benefit of priority of Russian Patent application 2006139128, filed November 7, 2006.

### BACKGROUND INFORMATION

### Field of the Disclosure

This disclosure relates in general to processes for the production of fluorinated internal olefins.

### Description of the Related Art

The refrigeration industry has been working for the past few decades to find replacement refrigerants for the ozone depleting chlorofluorocarbons (CFCs) and hydrochlorofluorocarbons (HCFCs) being phased out as a result of the Montreal Protocol. The solution for most refrigerant producers has been the commercialization of hydrofluorocarbon (HFC) refrigerants. The new HFC refrigerants, HFC-134a being the most widely used at this time, have zero ozone depletion potential and thus are not affected by the current regulatory phase-out as a result of the Montreal Protocol.

Further, environmental regulations may ultimately cause global phase-out of certain HFC refrigerants. Currently, the automobile industry is facing regulations relating to global warming potential (GWP) for refrigerants used in mobile air-conditioning. Therefore, there is a great current need to identify new refrigerants with reduced GWP for the automobile air-conditioning market. Should the regulations be more broadly applied in the future, an even greater need will be felt for low GWP refrigerants that can be used in all areas of the refrigeration and air-conditioning industry.

Currently proposed replacement refrigerants for HFC-134a include HFC-152a, pure hydrocarbons such as butane or propane, or "natural" refrigerants such as CO₂ or ammonia. Many of these suggested replacements are toxic, flammable, and/or have low energy efficiency. Therefore, new alternatives are constantly being sought.

Non-ozone depleting solvents with lower GWP than current HFC solvents are also constantly being sought. Among the current candidate refrigerants and solvents which are non-ozone depleting and which have lower GWP are hydrofluoroolefins. Some of these are lower boiling, lower molecular weight compounds such as the various isomers of tetrafluoropropene and pentafluoropropene. Other potentially useful compounds include higher molecular weight hydrofluoro-olefins, which may include olefins with terminal double bonds, and olefins with internal double bonds.

The prior art discloses the Lewis acid catalyzed addition of saturated compounds across the double bonds of various fluorinated-olefins, see for example WO 97/02227, US Patent No. 6,184,426 and US Patent Application Publication No. 2006/0258891.

### SUMMARY

A process is provided for the preparation of internal dihydrofluoroolefins of the formula RCH=CHC₂F₅, comprising reacting a fluorinated olefin of the formula RCH=CHF, wherein R is selected from perfluoroalkyl groups having from one to ten carbon atoms, and the said alkyl group is either an n-alkyl chain, a sec-alkyl chain, or an iso-alkyl chain, in the liquid phase with tetrafluoroethylene, in the presence of a Lewis acid catalyst, removing the Lewis acid catalyst and isolating the dihydrofluoroolefin.

Additionally provided is a method for the addition of terminal fluorinated olefins to tetrafluoroethylene.

The foregoing general description and the following detailed description are exemplary and explanatory only and are not restrictive of the invention, as defined in the appended claims.

### DETAILED DESCRIPTION

A process is provided for the preparation of internal dihydrofluoroolefins of the formula RCH=CHC₂F₅, reacting a fluorinated olefin of the formula RCH=CHF, wherein R is selected from perfluoroalkyl groups having from one to ten carbon atoms, and the said alkyl group is either an n-alkyl chain, a sec-alkyl chain, or an iso-alkyl chain, in the liquid phase with tetrafluoroethylene, in the presence of a Lewis acid catalyst, removing the Lewis acid catalyst and isolating the dihydrofluoroolefin. The process may further comprise an aqueous washing step to remove catalyst, and the washing step may comprise washing with a phosphate buffer. The process may be conducted at from about -10 °C to about 30 °C.

Many aspects and embodiments have been described above and are merely exemplary and not limiting. After reading this specification, skilled artisans appreciate that other aspects and embodiments are possible without departing from the scope of the invention.

Other features and benefits of any one or more of the embodiments will be apparent from the following detailed description, and from the claims. The detailed description first addresses Definitions and Clarification of Terms followed by the Description, and finally Examples.

### 1. Definitions and Clarification of Terms

Before addressing details of embodiments described below, some terms are defined or clarified.

As used herein, a Lewis acid catalyst is a compound which is an electrophile and which can accept a pair of electrons to form a coordinate covalent bond and act as a catalyst for the reaction.

In one embodiment, the present invention can generalty be described as a process for the preparation of dihydrofluoroolefins of the formula R^{f}CH=CHR'^{f}, wherein R^{f} and R'^{f} are perfluoroalkyl groups where the alkyl group of R^{f} contains from 1 to about 10 carbon atoms, and the alkyl group of R'^{f} contains 2 carbons. The alkyl chain of R^{f} is either a straight n-alkyl chain, a branched sec-alkyl chain such as CF₃CF₂C(CF₃)F-, or a branched iso-alkyl chain such as CF₃CF(CF₃)CF₂-. Among the compounds which can be prepared using the present method are 2,3-dihydro-1,1,1,4,4,5,5,5-octafluoropent-2-ene (F12E) (R^{f} = CF₃), 1,1,1,2,2,5,5,6,6,6-decafluoro-3-hexene (F22E) (R^{f} = C₂F₅) and 1,1,1,2,2,3,3,6,6,7,7,7-dodecafluoro-4-heptene (F23E) (R^{f} = n-C₃F₇). Unless otherwise indicated, throughout this application the fluoroolefins referred to herein refer to either the *Z*-, or *E*- isomers, or a mixture thereof.

In one embodiment, the dihydrofluoroolefins are prepared through reaction of, a fluorinated olefin compound with tetrafluoroethylene in the presence of a Lewis acid catalyst.

In one embodiment, the fluorinated olefin compounds are terminal fluorinated olefins of the structure RCH=CHF, and are either available commercially, or can be prepared by known methods in the art. The alkyl chain of R is either a straight n-alkyl chain, a branched sec-alkyl chain such as CF₃CF₂C(CF₃)F-, or a branched iso-alkyl chain such as CF₃CF(CF₃)CF₂-, having from one to ten carbon atoms. By way of example, CF₃CH=CHF (HFC-1234ze)(*Z*- and *E*- isomers), and CF₃CF₂CH=CHF (HFC-1336mce)(*Z*- and *E*- isomers) are readily obtained by dehydrofluorination of the corresponding hydrofluorocarbons.

In one embodiment, tetrafluoroethylene is added in essentially an equivalent molar amount to the fluorinated olefin compound. In another embodiment, from about 1.0 to about 1.05 moles of tetrafluoroethylene are added per mole of fluorinated olefin compound. Greater amounts of tetrafluoroethylene can lead to the formation of additional products arising from the addition of multiple equivalents of tetrafluoroethylene to form higher molecular weight fluoroolefins. In another embodiment, the molar ratio of tetrafluoroethylene to fluorinated olefin is less than 1:1. In yet another embodiment, the molar ratio of tetrafluoroethylene to fluorinated olefin is less than 0.60:1.

In one embodiment, the reaction is conducted in the liquid phase, in the presence of a Lewis acid catalyst such as SbF₅ or AlZ₃ where Z is one or more of Br, F or Cl, provided that Z cannot be entirely F. In one embodiment of the invention, the Lewis acid can be antimony pentafluoride or AlClₓF_{y} (mixed aluminum halide) where x plus y equals 3, and where x is from about 0.05 to about 2.95 and y is from about 2.95 to about 0.05. Details of the aluminum chlorofluoride catalyst preparation are described in US Patent 5,162,594. Since the catalysts are water sensitive, reagents and equipment should be dried before use.

In one embodiment, the temperature employed in the process of the instant invention typically ranges from about -10 °C to about 50 °C. In another embodiment, the temperature range is from about -10 °C to about 30 °C. In yet another embodiment, temperature range is from about -5 °C to about 25 °C.

Reaction time is not critical and typically ranges from 1 hour to 24 hours. In one embodiment, from 4 to 24 hours is usually sufficient. In some embodiments, when the reaction conditions are heterogeneous, some degree of agitation is often desirable.

The pressure employed in the reaction is not critical. In one embodiment, the reaction is normally run at pressure in the range of from about 0 to about 300 psig (101 kPa to 2169 kPa). Autogenous pressures are usually employed; however, the pressure should not be allowed to rise above 300 psig when using tetrafluoroethylene because of safety considerations.

In one embodiment, the product can be isolated by conventional means, for example aqueous washing to remove the Lewis acid catalyst, followed by fractionation. In another embodiment, to further ensure removal of any Lewis acid catalyst, the washing step can be conducted with an aqueous buffer solution, such as an aqueous phosphate buffer. In yet another embodiment, if further purification is required, the product of the process can be further purified via fractional distillation.

In general, the reaction proceeds with retention of stereochemistry about the double bond of the fluroolefin. Thus, as illustrated in example 2, starting with the *E*- isomer of 1,3,3,3-tetrafluoropropene produces the *E-*isomer of the product, F12E.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, use of "a" or "an" are employed to describe elements and components described herein. This is done merely for convenience and to give a general sense of the scope of the invention. This description should be read to include one or at least one and the singular also includes the plural unless it is obvious that it is meant otherwise. Group numbers corresponding to columns within the Periodic Table of the elements use the "New Notation" convention as seen in the CRC Handbook of Chemistry and Physics, 81st Edition (2000-2001).
Unless otherwise defined, all technical and scientific terms used herein have the same meaning as commonly understood by one of ordinary skill in the art to which this invention belongs. Although methods and materials similar or equivalent to those described herein can be used in the practice or testing of embodiments of the present invention, suitable methods and materials are described below. All publications, patent applications, patents, and other references mentioned herein are incorporated by reference in their entirety, unless a particular passage is cited. In case of conflict, the present specification, including definitions, will control. In addition, the materials, methods, and examples are illustrative only and not intended to be limiting.

### EXAMPLES

The concepts described herein will be further described in the following examples, which do not limit the scope of the invention described in the claims.

### Example 1

A 25 mL Hastelloy shaker tube was loaded with 2 g of antimony pentafluoride catalyst, closed, cooled in liquid nitrogen, evacuated and loaded with 10 g (0.087 mole) of 1,3,3,3-tetrafluoropropene and 9 g of (0.090 mole) of tetrafluoroethylene. The reaction vessel was allowed to warm and kept on a shaker at 20-25 °C at autogenous pressure for 24 hours. After 24 hours, the pressure tube was unloaded and 18 g of reaction was obtained, which was determined to contain 65% of CF₃CH=CHC₂F₅, and 27% of C₃F₇CH=CHC₂F₅ according to gas chromatography and ¹⁹F NMR. The reaction mixture was distilled to provide 10 g of CF₃CH=CHC₂F₅, (b.p. 30-33 °C) and 2.2 g of C₃F₇CH=CHC₂F₅ (b.p. 72-73 °C).

### Example 2

### Synthesis of 2,3-dihydro-1,1,1,4,4,5,5,5-octafluoropent-2-ene (CF₃CH=CHCF₂CF₃, F12E) and 3,4-dihydro-1,1,1,2,2,5,5,6,6,7,7,7-dodecafluoropent-3-ene (F23E)

To a 400 ml Hastelloy® shaker tube was added 8.5 gm (39.4 mmol) of SbF₅. The tube was cooled to about -34°C and 40 gm (351 mmol) of E-1,3,3,3-tetrafluoroprop-1-ene was condensed in. Then immediately at -34 °C, 20 gm (200 mmol) of tetrafluoroethylene was condensed in and the temperature increased to -3°C and the pressure increased to 109 psi. Within 30 minutes, the pressure dropped to 30 psi indicating a substantial reaction of the tetrafluoroethylene. After 24 more minutes, an additional 16 gm (160 mmol) of tetrafluoroethylene was added and the pressure increased to 132 psi, and after an additional 24 minutes, the pressure dropped to 6 psi. The mixture was shaken for an additional 17 hours and 22 minutes at or around room temperature. The product was transferred via vacuum to a stainless steel cylinder and neutralized with phosphate buffer (1M, pH=7) twice and then with water. Analysis by gas chromatography of the liquid layer showed 98.2% F12E and 0.85% F23E.

### Example 3

Still in a 400 ml Hastelloy® shaker tube, the above reaction was scaled up using 20 gm (92.6 mmol) of SbF₅, 100 gm (877 mmol) of E-1,3,3,3-tetrafluoroprop-1-ene and 90 gm (900 mmol) of tetrafluoroethylene in 30 gm increments. The product was treated as above and the liquid layer contained 80.3% F12E, 4.1% F23E and a variety of other compounds. This scaled up reaction was repeated again to in total yield 258 grams of crude material for distillation on a spinning band column using a high reflux ratio to yield 181 gm of E-F12E in high purity (bp = 29.4 - 29.5°C, 99.92% by gas chromatography area %, ¹⁹F: -66.5 (CF₃CH, m, 3F), -85.3 (CF₃, m 3F), -118.1 (CF₂, m, 2F); ¹H: 6.49 (m, 2H).

### Example 4

A 25 mL Hastelloy shaker tube is loaded with 1.6 g of antimony pentafluoride catalyst, closed, cooled in liquid nitrogen, evacuated and loaded with 11.5 g (0.070 mole) of 1,3,3,4,4,4-hexafluoro-1-butene and 7.2 g of (0.072 mole) of tetrafluoroethylene. The reaction vessel is allowed to warm and kept on a shaker at 20-25 °C at autogenous pressure for 24 hours. After 24 hours, the pressure tube is unloaded and 17.5 g of reaction was obtained, which is determined to contain 88% of CF₃CF₂CH=CHCF₂CF₃, and 6% of CF₃CF₂CF₂CF₂CH=CHCF₂CF₃ according to gas chromatography and ¹⁹F NMR. The reaction mixture is distilled to provide 11 g of CF₃CF₂CH=CHCF₂CF₃, (b.p._{~}51 °C) and 0.8 g of CF₃CF₂CF₂CF₂CH=CHCF₂CF₃ (b.p. ∼95 °C).

### Example 5

Seven grams of AlClₓF_{y}, as described in US 5157171, was loaded in a glove bag into a 400ml Hastelloy autoclave purged with nitrogen. The reactor was cooled in dry-ice and evacuated. A mixture of 1234ze and 245fa (130 gm, 70:30) was added to the reactor. TFE (30 gm, 0.3 mmol) was added at -30°C, and then in 10 min another addition of TFE (30 gm, 0.3 mmol) was made at -17.5°C. The increase in temperature was due to the exothermic nature of the reaction. After four minutes, the last portion of TFE (25gm, 0.25 mmol) was added at -3°C, and then the reaction proceeded for 8 hr. The crude product was distilled to give 96gm (.45 moles, 53%) of 2,3-dihydrooctafluoropentene-2.

Note that not all of the activities described above in the general description or the examples are required, that a portion of a specific activity may not be required, and that one or more further activities may be performed in addition to those described. Still further, the order in which activities are listed are not necessarily the order in which they are performed.

## Claims

1. A process for the preparation of dihydrofluoroolefins having a formula RCH=CHCF₂CF₃, comprising:
reacting a fluorinated olefin of the formula RCH=CHF, wherein R is a perfluorinated alkyl group having from one to ten carbon atoms, and the said alkyl group is either an n-alkyl chain, a sec-alkyl chain, or an iso-alkyl chain, in the liquid phase with tetrafluoroethylene in the presence of a Lewis acid catalyst;
removing the said Lewis acid catalyst; and
isolating the dihydrofluoroolefin.

2. A process as in claim 1 wherein the Lewis acid is removed in an aqueous washing step.

3. A process as in claim 2 wherein the aqueous washing step comprises washing the product with an aqueous phosphate buffer solution.

4. A process as in claim 1 wherein the reaction is conducted at from -10 °C to 30 °C.

5. A process as in claim 4 wherein the reaction is conducted at from -5 °C to 25 °C.

6. A process as in claim 1 wherein the tetrafluoroethylene is present at from an equivalent molar amount to a 5% molar excess relative to the said fluorinated olefin.

7. A process as in claim 1 wherein said fluorinated olefin is the *Z-*stereoisomer.

8. A process as in claim 7 wherein the said product dihydrofluoroolefin is the *Z*- stereoisomer.

9. A process as in claim 1 wherein said fluorinated olefin is the *E-*stereoisomer.

10. A process as in claim 9 wherein the said product dihydrofluoroolefin is the *E*- stereoisomer.

11. A process as in claim 1 wherein R is a trifluoromethyl group.

12. A process as in claim 1 wherein the Lewis acid is antimony pentafluoride.

13. A process as in claim 1 wherein the Lewis acid is aluminum chlorofluoride.

14. A process as in claim 1 wherein the dihydrofluoroolefin product is isolated by distillation.

## Patentansprüche

1. Verfahren zur Herstellung von Dihydrofluorolefinen, die die Formel RCH=CHCF₂CF₃ aufweisen, umfassend:
das Reagieren eines fluorierten Olefins der Formel RCH=CHF, wobei R eine perfluorierte Alkylgruppe ist, die ein bis zehn Kohlenstoffatome aufweist, und die Alkylgruppe entweder eine n-Alkylkette, eine sek-Alkylkette oder eine Isoalkylkette ist, in der flüssigen Phase mit Tetrafluorethylen in Gegenwart eines Lewis-Säurekatalysators;
das Entfernen des Lewis-Säurekatalysators; und
das Isolieren des Dihydrofluorolefins.

2. Verfahren nach Anspruch 1, wobei die Lewis-Säure in einem wässrigen Waschschritt entfernt wird.

3. Verfahren nach Anspruch 2, wobei der wässrige Waschschritt das Waschen des Produkts mit einer wässrigen Phosphatpufferlösung umfasst.

4. Verfahren nach Anspruch 1, wobei die Reaktion bei -10 °C bis 30 °C durchgeführt wird.

5. Verfahren nach Anspruch 4, wobei die Reaktion bei -5 °C bis 25 °C durchgeführt wird.

6. Verfahren nach Anspruch 1, wobei das Tetrafluorethylen in einer Menge von einer moläquivalenten Menge bis zu einem molaren Überschuss von 5 % mit Bezug auf das fluorierte Olefin vorliegt.

7. Verfahren nach Anspruch 1, wobei das fluorierte Olefin das Z-Stereoisomer ist.

8. Verfahren nach Anspruch 7, wobei das Produkt Dihydrofluorolefin das Z-Stereoisomer ist.

9. Verfahren nach Anspruch 1, wobei das fluorierte Olefin das E-Stereoisomer ist.

10. Verfahren nach Anspruch 9, wobei das Produkt Dihydrofluorolefin das E-Stereoisomer ist.

11. Verfahren nach Anspruch 1, wobei R eine Trifluormethylgruppe ist.

12. Verfahren nach Anspruch 1, wobei die Lewis-Säure Antimonpentafluorid ist.

13. Verfahren nach Anspruch 1, wobei die Lewis-Säure Aluminiumchlorfluorid ist.

14. Verfahren nach Anspruch 1, wobei das Dihydrofluorolefinprodukt durch Destillation isoliert wird.

## Revendications

1. Procédé de préparation de dihydro-fluoro-oléfines ayant une formule RCH=CHCF₂CF₃, comprenant:
la réaction d'une oléfine fluorée de la formule RCH=CHF,
dans laquelle R est un groupe alkyle perfluoré ayant de un à dix atome(s) de carbone, et ledit groupe alkyle est soit une chaîne *n*-alkyle, une chaîne *sec*-alkyle, soit une chaîne iso-alkyle, en phase liquide avec du tétrafluoroéthylène en présence d'un catalyseur de type acide de Lewis;
l'élimination dudit catalyseur du type acide de Lewis; et
l'isolement de la dihydro-fluoro-oléfine.

2. Procédé tel qu'énoncé selon la revendication 1, dans lequel l'acide de Lewis est retiré au cours d'une étape de lavage aqueux.

3. Procédé tel qu'énoncé selon la revendication 2, dans lequel l'étape de lavage aqueux comprend le lavage du produit avec une solution aqueuse de tampon phosphate.

4. Procédé tel qu'énoncé selon la revendication 1, dans lequel la réaction est conduite à -10°C jusqu'à 30°C.

5. Procédé tel qu'énoncé selon la revendication 4, dans lequel la réaction est conduite à -5°C jusqu'à 25°C.

6. Procédé tel qu'énoncé selon la revendication 1, dans lequel le tétrafluoroéthylène est présent en une quantité équivalente molaire de 5 % d'excès molaire par rapport à ladite oléfine fluorée.

7. Procédé tel qu'énoncé selon la revendication 1, dans lequel ladite oléfine fluorée est le stéréoisomère *Z*.

8. Procédé tel qu'énoncé selon la revendication 7, dans lequel ledit produit dihydro-fluoro-oléfine est le stéréoisomère *Z*.

9. Procédé tel qu'énoncé selon la revendication 1, dans lequel ladite oléfine fluorée est le stéréoisomère *E*.

10. Procédé tel qu'énoncé selon la revendication 9, dans lequel ledit produit dihydro-fluoro-oléfine est le stéréoisomère *E*.

11. Procédé tel qu'énoncé selon la revendication 1, dans lequel R est un groupe trifluorométhyle.

12. Procédé tel qu'énoncé selon la revendication 1, dans lequel l'acide de Lewis est le pentafluorure d'antimoine.

13. Procédé tel qu'énoncé selon la revendication 1, dans lequel l'acide de Lewis est le chlorofluorure d'aluminium.

14. Procédé tel qu'énoncé selon la revendication 1, dans lequel le produit dihydro-fluoro-oléfine est isolé par distillation.
